(19) Europäisches Patentamt
European Patent Office
Office européen des brevets

(11) **EP 1 733 717 B2**

(12) **NEW EUROPEAN PATENT SPECIFICATION**
After opposition procedure

(45) Date of publication and mention of the opposition decision:
**13.06.2012 Bulletin 2012/24**

(45) Mention of the grant of the patent:
**15.10.2008 Bulletin 2008/42**

(21) Application number: **06290913.0**

(22) Date of filing: **06.06.2006**

(51) Int Cl.:
***A61K 8/58*** $^{(2006.01)}$ ***A61K 8/55*** $^{(2006.01)}$
***A61K 8/41*** $^{(2006.01)}$ ***A61K 8/64*** $^{(2006.01)}$
***A61Q 5/12*** $^{(2006.01)}$ ***A61Q 5/02*** $^{(2006.01)}$

(54) **Aqueous carrier systems for water-insoluble materials**

Wässrige Trägersysteme für wasserunlösliche Materialien

Systemes excipients aqueux pour matières insolubles dans l' eau

(84) Designated Contracting States:
**AT BE BG CH CY CZ DE DK EE ES FI FR GB GR HU IE IS IT LI LT LU LV MC NL PL PT RO SE SI SK TR**

(30) Priority: **16.06.2005 US 154437**
**16.06.2005 US 154155**
**16.06.2005 US 154156**
**16.06.2005 US 154249**

(43) Date of publication of application:
**20.12.2006 Bulletin 2006/51**

(73) Proprietor: **L'Oréal**
**75008 Paris (FR)**

(72) Inventors:
- **Nguyen, Nghi**
**Edison, NJ 08820 (US)**
- **Cannell, David**
**Plainfield, NJ 07060 (US)**
- **Chong Espino, Cynthia**
**Princeton, NJ 08540 (US)**
- **Hashimoto, Sawa**
**Westfield, NJ 07090 (US)**
- **Barger, Katherine Natalie**
**Cranford, NJ 07016 (US)**
- **Lanzetta, Lydia**
**Scotch Plains, NJ 07076 (US)**

(74) Representative: **Dossmann, Gérard**
**Casalonga & Partners**
**Bayerstrasse 71-73**
**80335 München (DE)**

(56) References cited:
**EP-A- 1 402 881** **EP-A1- 1 402 881**
**FR-A1- 2 807 316** **US-A- 5 362 484**
**US-A- 5 362 484** **US-A- 6 024 952**
**US-A1- 2004 052 748** **US-A1- 2004 052 748**
**US-A1- 2004 170 587** **US-A1- 2004 170 587**
**US-B1- 6 602 494** **US-B1- 6 602 494**

- **"NEW COSMETIC FORMULATIONS" RESEARCH DISCLOSURE, MASON PUBLICATIONS, HAMPSHIRE, GB, no. 443, March 2001 (2001-03), pages 377-382, XP001126547 ISSN: 0374-4353**
- **GOTTSCHALK ET AL: 'International Cosmetic Ingredient Dictionary and Handbook', 2004, THE COSMETIC, TOILETRY AND FRAGRANCE ASSOCIATION, WASHINGTON DC, USA * page 1216 ***

EP 1 733 717 B2

## Description

### BACKGROUND OF THE INVENTION

The present invention relates to a novel carrier system based on a combination of at least one nonionic surfactant, having an HLB of at least 8, at least one anionic silicone, and at least one amphiphilic lipid chosen from phospholipids, fatty monoamine compounds, fatty quaternary amine compounds, and mixtures thereof. Such a carrier system allows water-insoluble materials to be incorporated into aqueous solutions.

Certain water-insoluble ingredients which are oftentimes desirable for the treatment of keratinous substrates are inherently difficult to incorporate into aqueous systems such as shampoos and conditioners without forming a traditional emulsion in either cream or lotion form. Moreover, many of these water-insoluble ingredients suppress lathering which makes the use of aqueous systems such as shampoos and body washes less desirable to consumers. Even in those aqueous systems which do employ these types of water-insoluble ingredients, their presence is minimal due to various performance drawbacks such as poor spreadability, foaming, removal and rinsing or, in the case of styling products, difficulties in removal via shampooing.

Also, when formulating clear to slightly limpid aqueous delivery systems for use in treating keratinous substrates, water-insoluble compounds do not lend themselves to being used therein, due to their inability to significantly associate with the water present in the system.

Thus, there remains a need for an aqueous delivery system which can carry water-insoluble materials while remaining both stable and clear, to slightly limpid, in appearance.

### SUMMARY OF THE INVENTION

In order to achieve these and other advantages, the present invention is drawn to a carrier composition as defined in claim 1.

Such a carrier composition, when combined with an aqueous phase, forms an aqueous delivery system which is both stable, and clear to slightly hazy/limpid in appearance.

In another embodiment, the present invention is also drawn to an aqueous delivery system which is both stable, and clear to slightly limpid in appearance, comprising the carrier composition as described herein combined with an aqueous phase.

In another embodiment, the present invention is also drawn to a process for making such an aqueous delivery system, as defined in claim 15.

Finally, in yet another embodiment, the present invention is drawn to a process for treating a keratinous substrate by contacting said substrate with an aqueous delivery system containing the above-disclosed carrier composition.

### DETAILED DESCRIPTION

Other than in the operating examples, or where otherwise indicated, all numbers expressing quantities of ingredients and/or reaction conditions are to be understood as being modified in all instances by the term "about".

The term "water-insoluble" means those compounds which are either completely or partially insoluble in water.

The term "carried" means that the aqueous delivery system containing the water-insoluble ingredients is both stable and clear, to slightly limpid, in appearance.

"Keratinous substrate" as defined herein may be human keratinous fiber, and may be chosen from, for example, hair, eyelashes, and eyebrows, as well as the stratum corneum of the skin and nails.

"At least one" as used herein means one or more and thus includes individual components as well as mixtures/combinations.

"Conditioning" as used herein means imparting to at least one keratinous fiber at least one property chosen from combability, manageability, moisture-retentivity, luster, shine, and softness. The state of conditioning is evaluated by measuring, and comparing, the ease of combability of the treated hair and of the untreated hair in terms of combing work (gm-in). See Examples 1-8.

"Formed from," as used herein, means obtained from chemical reaction of, wherein "chemical reaction," includes spontaneous chemical reactions and induced chemical reactions. As used herein, the phrase "formed from", is open ended and does not limit the components of the composition to those listed, *e.g.,* as component (i) and component (ii). Furthermore, the phrase "formed from" does not limit the order of adding components to the composition or require that the listed components (e.g., components (i) and (ii)) be added to the composition before any other components.

"Hydrocarbons," as used herein, include alkanes, alkenes, and alkynes, wherein the alkanes comprise at least one carbon, and the alkenes and alkynes each comprise at least two carbons; further wherein the hydrocarbons may be chosen from linear hydrocarbons, branched hydrocarbons, and cyclic hydrocarbons; further wherein the hydrocarbons

may optionally be substituted; and further wherein the hydrocarbons may optionally further comprise at least one heteroatom intercalated in the hydrocarbon chain.

"Silicone compound," as used herein, includes, for example, silica, silanes, silazanes, siloxanes, and organosiloxanes; and refers to a compound comprising at least one silicon; wherein the silicone compound may be chosen from linear silicone compounds, branched silicone compounds, and cyclic silicone compounds; further wherein the silicone compound may optionally be substituted; and further wherein the silicone compound may optionally further comprise at least one heteroatom intercalated in the silicone chain, wherein the at least one heteroatom is different from the at least one silicon.

"Substituted," as used herein, means comprising at least one substituent. Non-limiting examples of substituents include atoms, such as oxygen atoms and nitrogen atoms, as well as functional groups, such as hydroxyl groups, ether groups, alkoxy groups, acyloxyalkyl groups, oxyalkylene groups, polyoxyalkylene groups, carboxylic acid groups, monoamine groups, acylamino groups, amide groups, halogen containing groups, ester groups, thiol groups, sulphonate groups, thiosulphate groups, siloxane groups, and polysiloxane groups. The substituent(s) may be further substituted.

"Ethylene oxide group" as defined herein refers to a group of formula $-CH_2CH_2-O-$.

"Propylene oxide group" as defined herein includes groups of formula $-CH_2CH_2CH_2-O-$, groups of formula $(CH_3)CHCH_2-O-$, and groups of formula $-CH_2\ (CH_3)CH-O-$.

"Polymers," as defined herein, include homopolymers and copolymers formed from at least two different types of monomers.

Advantageously, the present invention allows water-insoluble materials or ingredients to be carried in an aqueous solution. No alcohol is required to render the system stable and clear, to slightly limpid, in appearance.

The carrier composition of the invention is easy to formulate and gentle on the hair, skin, or eyelashes because the surfactants used therein are generally mild.

The compositions and delivery systems of the present invention readily deliver water-insoluble ingredients to the targeted keratinous substrate. Accordingly, these compositions and delivery systems can be used in hair shampoos, conditioners, hair dyeing compositions, including oxidative dyes and bleaches, permanent waving compositions, curl relaxing compositions, hair setting compositions, bath and body products, sunscreens, cosmetics, skin moisturizers, and the like.

These systems can also be used to deliver active water-insoluble pharmaceutical ingredients, particularly in topical applications. Such systems could further help protect against oxidation and rancidity by protecting sensitive ingredients in pharmaceuticals or foods.

Without being bound to a particular theory, the inventors believe that the aqueous delivery system is in the form of a microemulsion whereby the anionic silicone forms an ion pair or pseudo-soap with said amphiphilic lipid which is then coupled to the aqueous solution as a mixed micelle (or other organized structure) by the nonionic surfactant. This structure is believed to be of sufficient stability and size to provide lipophilic regions which carry the water-insoluble ingredients. These microemulsions range from pourable liquids to firm ringing gels.

According to the present invention, said amphiphilic lipid can be chosen from phospholipids, such as conventional organic phospholipids.

Particularly preferred organic phospholipids include lecithins. Lecithins are mixtures of phospholipids, i.e., of diglycerides of fatty acids linked to an ester of phosphoric acid. Preferred lecithins are diglycerides of stearic, palmitic, and oleic acids linked to the choline ester of phosphoric acid. Lecithin is usually defined either as pure phosphatidyl cholines or as crude mixtures of phospholipids which include phosphatidyl choline, phosphatidyl serine, phosphatidyl ethanolamine, phosphatidyl inositol, other phospholipids, and a variety of other compounds such as fatty acids, triglycerides, sterols, carbohydrates, and glycolipids.

The lecithin used in the present invention may be present in the form of a liquid, powder, or granules. Lecithins useful in the invention include, but are not limited to, soy lecithin and hydroxylated lecithin. For example, ALCOLEC S is a fluid soy lecithin, ALCOLEC F 100 is a powder soy lecithin, and ALCOLEC Z3 is a hydroxylated lecithin, all of which are available from the American Lecithin Company.

Other than lecithins, additional examples of phospholipids which may be useful in the present invention include, but are not limited to, multifunctional biomimetic phospholipids. For example, the following multifunctional biomimetic phospholipids manufactured by Uniqema Industries may be useful: PHOSPHOLIPID PTC, PHOSPHOLIPID CDM, PHOSPHOLIPID SV, PHOSPHOLIPID GLA, and PHOSPHOLIPID EFA.

According to the present invention, said amphiphilic lipid can be chosen from conventional fatty monoamine compounds.

Fatty monoamine compounds are those which have at least one hydrocarbon group with from 6 to 22 carbon atoms. Primary, secondary, and tertiary fatty monoamines are useful. Particularly useful are tertiary amido amines having an alkyl group of from about 6 to about 22 carbons. Exemplary tertiary amido amines include: stearamidopropyldimethylamine, stearamidopropyldiethylamine, stearamidoethyldiethylamine, stearamidoethyldimethylamine, palmitamidopropyldimethyl amine, palmitamidopropyldiethylamine, palmitamidoethyldiethylamine, palmitamidoethyldimethylamine, behenamidopropyldimethylamine, behenamidopropyldiethylamine, behenamidoethyldiethylamine, behenamidoethyldimethylamine, arachnidamidopropyldimethylamine, arachidamidopropyldiethylamine, arachidamidoethyldiethylamine, arachidamidoethyldimethylamine, diethylaminoethylstearamide. Also useful are dimethylstearamine, dimethylsoyamine, soyamine, myristylamine, tridecylamine, ethylstearylamine, N-tallowpropane diamine, hydroxylated, ethoxylated or propoxylated fatty amines such as ethoxylated stearylamine, dihydroxyethylstearylamine, and arachidylbehenylamine. Useful amines in the present invention are disclosed in U.S. Pat. No. 4,275,055.

According to the present invention, said amphiphilic lipid can be chosen from conventional fatty quaternary amine compounds, such as fatty quaternary amine compounds containing at least one fatty chain having from about 6 to about 22 carbon atoms.

The anion of the quaternary ammonium compound can be a common ion such as chloride, ethosulfate, methosulfate, acetate, bromide, lactate, nitrate, phosphate, or tosylate and mixtures thereof. The long chain alkyl groups can include additional or replaced carbon or hydrogen atoms or ether linkages. Other substitutions on the quaternary nitrogen can be hydrogen, benzyl or short chain alkyl or hydroxyalkyl groups such as methyl, ethyl, hydroxymethyl or hydroxyethyl, hydroxypropyl or combinations thereof.

Examples of fatty quaternary ammonium compounds include but are not limited to: behentrimonium chloride, cocotrimonium chloride, cethethyldimonium bromide, dibehenyldimonium chloride, dihydrogenated tallow benzylmonium chloride, disoyadimonium chloride, ditallowdimonium chloride, hydroxycetyl hydroxyethyl dimonium chloride, hydroxyethyl behenamidopropyl dimonium chloride, hydroxyethyl Cetyldimonium chloride, hydroxyethyl tallowdimonium chloride, myristalkonium chloride, PEG-2 oleamonium chloride, PEG-5 Stearmonium chloride, PEG-15 cocoyl quaternium 4, PEG-2 stearalkonium 4, lauryltrimonium chloride; Quaternium-16; Quaternium-18, lauralkonium chloride, olealkonium chloride, cetylpyridinium chloride, Polyquaternium-5, Polyquaternium-6, Polyquaternium-7, Polyquaternium-10, Polyquaternium-22, Polyquaternium-37, Polyquaternium-39, Polyquaternium-47, cetyl trimonium chloride, dilauryldimonium chloride, cetalkonium chloride, dicetyldimonium chloride, soyatrimonium chloride, stearyl octyl dimonium methosulfate, behentrimonium methosulfate (18-MEA), stearalkonium chloride, and mixtures thereof. Other quaternary ammonium compounds are listed in the CTFA Cosmetic Ingredient Handbook, First Edition, on pages 41-42, incorporated herein by reference.

According to the present invention, said amphiphilic lipid is preferably used in an amount of from greater than 0% to 30% by weight, preferably from greater than 0% to 10% by weight, and more preferably from greater than 0% to 5% by weight, based on the weight of the carrier composition as a whole.

Preferably, the carrier composition of the present invention, when combined with water, forms a clear solution, though the purpose of the invention is achieved just as effectively with a slightly cloudy/limpid solution.

In general, nonionic surfactants having a Hydrophilic-Lipophilic Balance (HLB) of from 8 to 20, are contemplated for use by the present invention.

Nonionic surfactants useful herein include alkoxylated derivatives of the following: fatty alcohols, alkyl phenols, fatty acids, fatty acid esters and fatty acid amides, wherein the alkyl chain is in the C12-C50 range, preferably in the C16-C40 range, more preferably in the C24 to C40 range, and having from about 1 to about 110 alkoxy groups. The alkoxy groups are selected from the group consisting of C2-C6 oxides and their mixtures, with ethylene oxide, propylene oxide, and their mixtures being the preferred alkoxides. The alkyl chain may be linear, branched, saturated, or unsaturated. Of these alkoxylated non-ionic surfactants, the alkoxylated alcohols are preferred, and the ethoxylated alcohols and propoxylated alcohols are more preferred. The alkoxylated alcohols may be used alone or in mixtures thereof. The alkoxylated alcohols may also be used in mixtures with those alkoxylated materials disclosed herein-above.

Other representative examples of such ethoxylated fatty alcohols include laureth-3 (a lauryl ethoxylate having an average degree of ethoxylation of 3), laureth-23 (a lauryl ethoxylate having an average degree of ethoxylation of 23), ceteth-10 (a cetyl alcohol ethoxylate having an average degree of ethoxylation of 10) steareth-10 (a stearyl alcohol ethoxylate having an average degree of ethoxylation of 10), and steareth-2 (a stearyl alcohol ethoxylate having an average degree of ethoxylation of 2), steareth-100 (a stearyl alcohol ethoxylate having an average degree of ethoxylation of 100), beheneth-5 (a behenyl alcohol ethoxylate having an average degree of ethoxylation of 5), beheneth-10 (a behenyl alcohol ethoxylate having an average degree of ethoxylation of 10), and other derivatives and mixtures of the preceding.

Also available commercially are Brij® nonionic surfactants from ICI Specialty Chemicals, Wilmington, Del. Typically, Brij® is the condensation products of aliphatic alcohols with from about 1 to about 54 moles of ethylene oxide, the alkyl chain of the alcohol being typically a linear chain and having from about 8 to about 22 carbon atoms, for example, Brij 72 (i.e., Steareth-2) and Brij 76 (i.e., Steareth-10).

Also useful herein as nonionic surfactants are alkyl glycosides, which are the condensation products of long chain alcohols, e.g. C8-30 alcohols, with sugar or starch polymers. These compounds can be represented by the formula (S)n --O--R wherein S is a sugar moiety such as glucose, fructose, mannose, galactose, and the like; n is an integer of from about 1 to about 1000, and R is a C8-30 alkyl group. Examples of long chain alcohols from which the alkyl group can be derived include decyl alcohol, cetyl alcohol, stearyl alcohol, lauryl alcohol, myristyl alcohol, oleyl alcohol, and the

like. Preferred examples of these surfactants are alkyl polyglucosides wherein S is a glucose moiety, R is a C8-20 alkyl group, and n is an integer of from about 1 to about 9. Commercially available examples of these surfactants include decyl polyglucoside (available as APG® 325 CS) and lauryl polyglucoside (available as APG® 600CS and 625 CS), all the above-identified polyglucosides APG® are available from Cognis, Ambler, Pa. Also useful herein are sucrose ester surfactants such as sucrose cocoate and sucrose laurate.

Other nonionic surfactants suitable for use in the present invention are glyceryl esters and polyglyceryl esters, including but not limited to, glyceryl monoesters, preferably glyceryl monoesters of C16-C22 saturated, unsaturated and branched chain fatty acids such as glyceryl oleate, glyceryl monostearate, glyceryl monoisostearate, glyceryl monopalmitate, glyceryl monobehenate, and mixtures thereof, and polyglyceryl esters of C16-C22 saturated, unsaturated and branched chain fatty acids, such as polyglyceryl-4 isostearate, polyglyceryl-3 oleate, polyglyceryl-2 sesquioleate, triglyceryl diisostearate, diglyceryl monooleate, tetraglyceryl monooleate, and mixtures thereof.

Also useful herein as nonionic surfactants are sorbitan esters. Preferable are sorbitan esters of C16-C22 saturated, unsaturated and branched chain fatty acids. Because of the manner in which they are typically manufactured, these sorbitan esters usually comprise mixtures of mono-, di-, tri-, etc. esters. Representative examples of suitable sorbitan esters include sorbitan monooleate (e.g., SPAN® 80), sorbitan sesquioleate (e.g., Arlacel® 83 from ICI Specialty Chemicals, Wilmington, Del.), sorbitan monoisostearate (e.g., CRILL® 6 from Croda, Inc., Parsippany, N.J.), sorbitan stearates (e.g., SPAN® 60), sorbitan trioleate (e.g., SPAN® 85), sorbitan tristearate (e.g., SPAN® 65), sorbitan dipalmitates (e.g., SPAN® 40), and sorbitan isostearate. Sorbitan monoisostearate and sorbitan sesquioleate are particularly preferred emulsifiers for use in the present invention.

Also suitable for use herein are alkoxylated derivatives of glyceryl esters, sorbitan esters, and alkyl polyglycosides, wherein the alkoxy groups is selected from the group consisting of C2-C6 oxides and their mixtures, with ethoxylated or propoxylated derivatives of these materials being the preferred. Nonlimiting examples of commercially available ethoxylated materials include TWEEN® (ethoxylated sorbitan mono-, di- and/or tri-esters of C12 to C18 fatty acids with an average degree of ethoxylation of from about 2 to about 20).

Preferred nonionic surfactants are those formed from a fatty alcohol, a fatty acid, or a glyceride with a $C_4$ to $C_{36}$ carbon chain, preferably a $C_{12}$ to $C_{18}$ carbon chain, more preferably a $C_{16}$ to $C_{18}$ carbon chain, derivatized to yield an HLB of at least 8. HLB is understood to mean the balance between the size and strength of the hydrophilic group and the size and strength of the lipophilic group of the surfactant. Such derivatives can be polymers such as ethoxylates, propoxylates, polyglucosides, polyglycerins, polylactates, polyglycolates, polysorbates, and others that would be apparent to one of ordinary skill in the art. Such derivatives may also be mixed polymers of the above, such as ethoxylate/propoxylate species, where the total HLB is preferably greater than or equal to 8. Preferably the nonionic surfactants contain ethoxylate in a molar content of from 10-25, more preferably from 10-20 moles.

The nonionic surfactant will typically be present in the composition in an amount of from greater than 0% to 70% by weight, preferably from greater than 0% to 40% by weight, and more preferably from greater then 0% to 20% by weight, based on the weight of the carrier composition as a whole.

The anionic silicones used in the present invention include silicone carboxylates and silicone phosphates.

Suitable silicone carboxylates may be chosen from water soluble silicone compounds comprising at least one carboxylic acid group, oil soluble silicone compounds comprising at least one carboxylic acid group, water-dispersible silicone compounds comprising at least one carboxylic acid group, and silicone compounds comprising at least one carboxylic acid group which are soluble in organic solvents. In one embodiment, the at least one silicone compound comprising at least one carboxylic acid group further comprises at least one alkoxylated chain, wherein the at least one alkoxy group may be chosen from terminal alkoxy groups, pendant alkoxy groups, and alkoxy groups which are intercalated in the skeleton of the at least one silicone compound. Non-limiting examples of at least one alkoxy group include ethylene oxide groups and propylene oxide groups.

The at least one carboxylic acid group may be chosen from terminal carboxylic acid groups and pendant carboxylic acid groups. Further, the at least one carboxylic acid may be chosen from carboxylic acid groups in free acid form, i.e., -COOH, and carboxylic acid groups in salt form, i.e., -COOM, wherein M may be chosen from inorganic cations, such as, for example, potassium cations and sodium cations, and organic cations.

In one embodiment, the at least one silicone compound comprising at least one carboxylic acid group is chosen from silicone compounds of formula (I) and salts thereof:

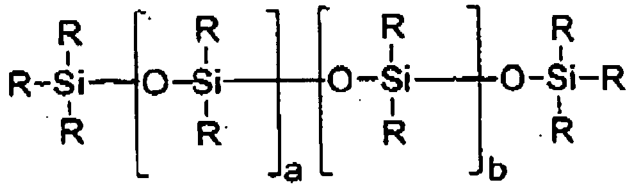

(I)

wherein: a is an integer ranging from 1 to 100; b is an integer ranging from 0 to 500; R, which may be identical or different, are each chosen from optionally substituted hydrocarbon groups comprising from 1 to 9 carbon atoms, optionally substituted phenyl groups, and groups of formula (II):

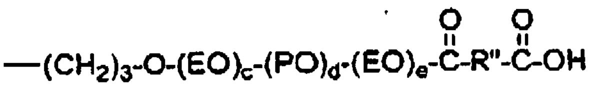

(II)

wherein: c, d, and e, which may be identical or different, are each integers ranging from 0 to 20; EO is an ethylene oxide group; PO is a propylene oxide group; and R" is chosen from optionally substituted divalent hydrocarbons, such as alkylene groups and alkenylene groups comprising from 2 to 22 carbon atoms, and optionally substituted divalent aromatic groups, such as groups of formula (III):

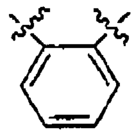

(III);

and groups of formula (IV):

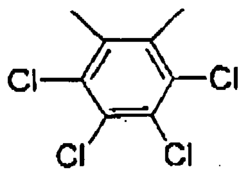

(IV);

with the proviso that at least one of the R groups is chosen from groups of formula (II) and with the further proviso that when only one of the R groups is chosen from groups of formula (II), the other R groups are not all methyl groups.

Non-limiting examples of the at least one silicone compound include those commercially available from Noveon under the name Ultrasil® CA-1 Silicone and Ultrasil ®CA-2 Silicone, both of which correspond to formula (V) below. This silicone carboxylate is sold in the free acid form as an emulsifier and dispersing aid for complexing fatty cationic amines and quaternary amines. Thus, in one embodiment, the at least one silicone compound is chosen from silicone compounds of formula (V) and salts thereof:

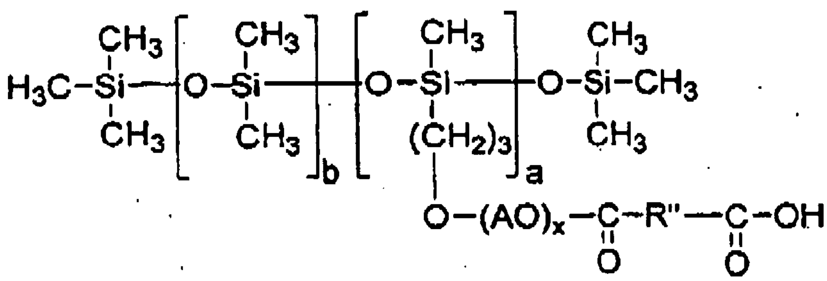

(V)

wherein: a is an integer ranging from 1 to 100; b is an integer ranging from 0 to 500; AO is chosen from groups of formula (VI):

$$-(EO)_c-(PO)_d-(EO)_e- \qquad (VI)$$

wherein: c, d, and e, which may be identical or different, are each integers ranging from 0 to 20; EO is an ethylene oxide group; and PO is a propylene oxide group; x is an integer ranging from 0 to 60; R" is chosen from optionally substituted divalent hydrocarbons, such as alkylene groups and alkenylene groups comprising from 2 to 22 carbon atoms, and optionally substituted divalent aromatic groups, such as groups of formula (III) :

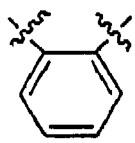

(III);

and groups of formula (IV):

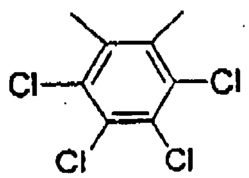

(IV).

Non-limiting examples of the at least one silicone compound include those described in U.S. Patent Nos. 5,248,783 and 5,739,371, the disclosures of which are incorporated herein by reference, and which are silicone compounds of formula (I).

Suitable silicone phosphates may be chosen from water-soluble silicone compounds comprising at least one phosphate group, oil soluble silicone compounds comprising at least one phosphate group, water-dispersible silicone compounds comprising at least one phosphate group, and silicone compounds comprising at least one phosphate group which are soluble in organic solvents.

In one embodiment, the at least one silicone compound comprising at least one phosphate group further comprises at least one alkoxylated chain, wherein the at least one alkoxy group may be chosen from terminal alkoxy groups, pendant alkoxy groups, and alkoxy groups which are intercalated in the skeleton of the at least one silicone compound. Non-limiting examples of at least one alkoxy group include ethylene oxide groups ("EO" = $-CH_2-CH_2-O-$) and propylene oxide groups ("PO" = $C_3H_6O$).

The at least one phosphate group may be chosen from terminal phosphate groups and pendant phosphate groups. Further, the at least one phosphate group may be chosen from groups of formula $-O-P(O)(OH)_2$, groups of formula -O-P(O) (OH) (OR) , and groups of formula $-O-P(O)(OR)_2$, wherein R may be chosen from H, inorganic cations, and organic cations. Non-limiting examples of inorganic cations include alkali metals, such as, for example, potassium lithium, and sodium. A non-limiting example of organic cations is at least one additional silicone compound which may be identical to or different from the at least one silicone compound bonded to the other oxygen of the phosphate group.

In one embodiment, the at least one silicone compound comprising at least one phosphate group is chosen

from silicone compounds of formula (VII):

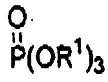 (VII)

wherein $R^1$, which may be identical or different, are each chosen from H, organic cations, inorganic cations, optionally substituted hydrocarbons (such as alkyl groups and alkenyl groups comprising from 1 to 22 carbon atoms), optionally substituted aromatic groups; groups of formula (VIII) and salts thereof:

$CH_3(CH_2)_x$-O-$(EO)_c$-$(PO)_d$-$(EO)_e$-$CH_2CH_2$- (VIII)

wherein: c, and d, which may be identical or different, are each integers ranging from 0 to 20; e is an integer ranging from 0 to 19; and x is an integer ranging from 0 to 21; groups of formula (IX) and salts thereof:

HO-$(EO)_c$-$(PO)_d$-$(EO)_e$-$(CH_2)_x$- (IX)

wherein: c, d, and e, which may be identical or different, are each integers ranging from 0 to 20; and x is an integer ranging from 0 to 21; and groups of formula (X) and salts thereof:

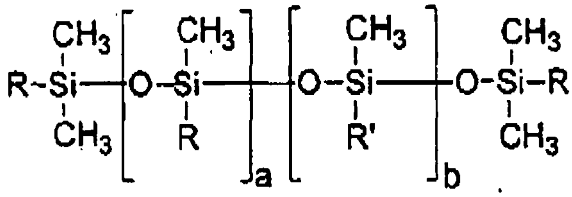 (X)

wherein: a is an integer ranging from 0 to 200; b is an integer ranging from 0 to 200; R', which may be identical or different, are each chosen from optionally substituted hydrocarbons, such as alkyl groups and alkenyl groups comprising from 1 to 22 carbon atoms, optionally substituted aromatic groups, groups of formula (IX) as defined above and salts thereof; and R, which may be identical or different, are each chosen from optionally substituted hydrocarbons, such as alkyl groups and alkenyl groups comprising from 1 to 22 carbon atoms, optionally substituted aromatic groups, optionally substituted divalent hydrocarbons, such as alkylene groups and alkenylene groups comprising from 1 to 22 carbon atoms, optionally substituted divalent aromatic groups, groups of formula (IX) as defined above and salts thereof, and groups of formula (XI):

-$(EO)_c$-$(PO)_d$-$(EO)_e$-$(CH_2)_3$- (XI)

wherein:

the $(CH_2)_3$ end is bonded to the silicon of the compound of formula (X) and the (EO) or (PO) end, if present, is bonded to the oxygen of the compound of formula (VII); c, d, and e, which may be identical or different, are each integers ranging from 0 to 20; EO is an ethylene oxide group; and PO is a propylene oxide group; and with the proviso that at least one R is chosen from groups of formula (XI) and salts thereof; and with the further proviso that at least one $R^1$ is chosen from groups of formula (X) and salts thereof and at least one other $R^1$ is chosen from H, organic cations, and inorganic cations.

Non-limiting examples of the inorganic cations include alkali metals, such as potassium, lithium, and sodium. Non-limiting examples of the at least one silicone compound include those commercially available from Phoenix Chemical, Inc. of New Jersey under the name of Pecosil®, such as Pecosil® PS-100, Pecosil® PS-112, Pecosil® PS-150, Pecosil® PS-200, Pecosil® WDS-100, Pecosil® WDS-200, Pecosil® PS-100 B, and Pecosil® PS-100 K and those commercially available from Siltech under the name Silphos A-100 and Silphos A-150. Other non-limiting examples of the at least one silicone compound include those described in U.S. Patent Nos. 5,070,171, 5,093,452, and 5,149,765 the disclosures of which are incorporated herein by reference.

A particularly preferred anionic silicone is Dimethicone PEG-8 phosphate, commercially available from Noveon under the tradename Ultrasil PE-100.

The anionic silicone is present in the composition in an amount ranging from greater than 0 to 50% by weight, preferably from greater than 0 to 30% by weight, and more preferably from greater than 0 to 15% by weight, based on the weight of the carrier composition as a whole.

It has surprisingly been found that the carrier composition of the present invention facilitates the formulation of an aqueous delivery system capable of carrying up to 10% by weight, based on the weight of the carrier composition, of water-insoluble ingredients. The resultant aqueous delivery system is both stable, and clear to slightly hazy/limpid in appearance.

Water-insoluble materials or ingredients include, but are not limited to, the following:

(1) Lipophilic "ingredients" or "materials" such as silicones, oil-soluble vitamins such as vitamin E and vitamin A, sunscreens, ceramides and natural oils. The lipophilic ingredients may be in the form of sunscreens, bacteriostats, moisturizers, colors, topical pharmaceuticals and the like. Preferred lipophilic ingredients include: vitamin E, vitamin E acetate, vitamin A palmitate, olive oil, mineral oil, 2-oleamido-1,3-octadecanediol, octylmethoxy cinnamate, octyl salicylate, and silicones such as dimethicone, cyclomethicone, phenyl trimethicone, dimethiconol, dimethicone copolyol, aminosilicone and laurylmethicone copolyol. The lipophilic ingredients will, for example, moisturize or condition the skin, hair, and/or eyelashes and leave behind no oily feel.

(2) Water-insoluble polymers, resins, and latexes, wherein the polymers and resins include but are not limited to those containing carboxyl moieties, such as acrylates and other carboxy polymers.

Preferred water-insoluble ingredients for use in the present invention include silicones ranging from low molecular weight fluids to high molecular weight gums; hydrocarbons such as mineral oil, petrolatum, paraffins, iso-paraffins, aromatic hydrocarbons, and the like; plant oils such as olive, avocado, coconut, and the like; fatty acids; fatty esters; fatty alcohols; and fatty waxes.

In another aspect, the present invention relates to an aqueous delivery system comprising the carrier composition and an aqueous phase.

The carrier composition is present in an amount sufficient to allow the at least one water-insoluble material to be incorporated into the aqueous system. The amount sufficient for incorporation may vary depending on the type of the final composition in which the aqueous delivery system is used; for example, shampoo and mascara formulations require a lower concentration of the carrier composition than do conditioner, deep treatment, bleach, permanent wave, dye, and relaxant compositions.

The aqueous phase of the delivery system can contain additional ingredients such as anionic surfactants, organic salts, inorganic salts, proteins, hair dyes, water-soluble polymers, quaternary ammonium compounds, complex and simple carbohydrates, amino acids, preservatives and fragrances.

In another embodiment, the present invention is drawn to a process for treating keratinous substances such as, but not limited to, hair, skin, or eyelashes by contacting the keratinous substance with the above-disclosed aqueous delivery system. The term treating in the context of this invention includes, but is not limited to, shampooing, conditioning, dyeing, bleaching, permanent waving, relaxing, setting, moisturizing, and making-up, for example, applying mascara or foundation.

As mentioned previously, the carrier composition and aqueous delivery system of the present invention can be used as an ingredient itself in, for example, shampoos, conditioners (rinse-off and leave-in), deep treatments for hair, body washes, bath gels, hair dyeing compositions, permanent wave formulations, relaxers, make-up preparations, particularly mascara and foundation, and skin creams or lotions.

The aqueous delivery systems of the invention can be further associated, in the hair products described above, with proteins including hydrolyzed soy protein, lauryldimonium hydrolyzed soy protein (cationic Soya protein) and wheat amino acids. The proteins could also include corn, wheat, milk, or silk proteins, collagens, keratins, or others. Furthermore, taurine and arginine hydrochloride may be associated therein to maximize protein binding to the keratinous substrate. Cationic proteins or proteins in general may be stabilizers for the aqueous delivery system and enhance its delivery by changing the charge of the aqueous delivery system. The skin and the hair attract cationic ingredients, and proteins are generally substantive to these tissues.

Other ingredients in the aqueous delivery system may include cationic polymers, such as polyquaternium 4, polyquaternium 6, polyquaternium 7, polyquaternium 10, polyquaternium 11, polyquaternium 16, polyquaternium 22, and polyquaternium 32, cationic conditioners, such as quaternium 27, behenamidopropyl PG-dimonium chloride, hydroxyethyl tallowdimonium chloride, hexadimethrine chloride, stearalkonium chloride, and cetrimonium chloride, isoparaffins, sodium chloride, propylene glycol, preservatives such as phenoxyethanol, methylparaben, ethylparaben, and propylparaben, pH adjusters such as phosphoric acid, humectants such as trehalose, and emollients such as octyldodecanol. Many other examples of materials from the classes listed above would be readily known to one of ordinary skill in the art.

Further, shampoos, conditioners, and deep treatments within the scope of the present invention may be used on hair which has been treated, e.g., with color (dye or bleach) or chemicals (permanent wave or straightening), or which is dry or fine and show significant substantivity for the hair.

The invention will be further clarified by the following examples, which are intended to be illustrative of the

invention, but not limiting thereof.

EXAMPLES

Example 1: amphiphilic lipid (a) chosen from phospholipids

General procedure: The general procedure for formulating carrier system is as follows: First, add deionized water to the beaker. Begin mixing at high speed. Next, slowly disperse lecithin. Mix well until lecithin is free of clumps. Then reduce mixing speed to moderate speed, and begin heating the batch to 80°C. At 75°C, add Procetyl AWS (INCI: PPG-5 Ceteth-10) and Ultrasil PE-100 (silicone phosphate). Mix well. At 80°C, add active ingredient. Mix well until formula is clear, maintaining at 80°C. Cool to room temperature. If necessary, pour at 75°C since formula may gel up around 70°C.

Example 1a: the carrier system

Following the general procedure, a series of solutions were made as depicted in Table 1a. The results show that a complete carrier system is needed to form a clear, stable solution that contains a water insoluble silicone.

Table 1a. Experiments showing a clear, stable carrier system

| Combinations of ingredients | Appearance |
|---|---|
| Water 46g + Lecithin 5g + DC 200 (300,000cst) 4g | Hazy |
| Water 46g + Procetyl AWS 25g + DC 200 (300,000cst) 4g | Hazy |
| Water 46g + Silicone Phosphate 20g + DC 200 (300,000cst) 4g | Hazy |
| Water 46g + Lecithin 5g + Procetyl AWS 25g + DC 200 (300,000cst) 4g | Hazy |
| Water 46g + Lecithin 5g + Silicone Phosphate 20g + DC 200 (300,000cst) 4g | Hazy |
| Water 46g + Procetyl AWS 25g + Silicone Phosphate 20g + DC 200 (300,000cst) 4g | Hazy |
| Water 46g + Lecithin 5g + Procetyl AWS 25g + Silicone Phosphate 20g + DC 200 (300,000cst) 4g | Clear |

Example 1b: the carrier system with different silicones

The carrier system is able to carry different types of silicones, such as DC 200 (INCI: dimethicone), DC 556 (INCI: phenyltrimethicone), and DC 5562 (INCI: Bis-hydroxyethoxypropyl dimethicone). When formulated using the general procedure as described above, clear, stable systems are obtained, as shown in table 1b.

Table 1b. Carrier system with different types of silicones

| | A | B | C | D | E |
|---|---|---|---|---|---|
| D.I. Water | 46% | 46% | 46% | 46% | 46% |
| Lecithin | 5% | 5% | 5% | 5% | 5% |
| PPG-5 Ceteth-20 | 25% | 25% | 25% | 25% | 25% |
| Silicone phosphate | 20% | 20% | 20% | 20% | 20% |
| Silicones | 4% (DC 200 (1000cst)) | 4% (DC 200 (60,000cst)) | 4% (DC 200 (300,000cst)) | 4% (DC 556) | 4% (DC 5562 Carbinol fluid) |

Furthermore, the above formula D is dilutable in water and in a shampoo base (25% TEA Lauryl sulfate, 10% Cocamidopropyl Betaine, and 65% DI Water).

Example 1c: the carrier system with different oils

The carrier system is able to carry different types of oils, such as mineral oil and tea tree oil. When formulated using the general procedure as described in above, clear, stable systems are obtained as shown in table 1c.

Table 1c. Carrier system with different types of oils

| | A | B |
|---|---|---|
| D.I. Water | 46% | 46% |
| Lecithin | 5% | 5% |
| PPG-5 Ceteth-20 | 25% | 25% |
| Silicone Phosphate | 20% | 20% |
| Oils | 4% (Mineral oil ) | 4% (Tea tree oil) |

Furthermore, the above formulas A and B are dilutable in water and in a shampoo base (25% TEA Lauryl sulfate, 10% Cocamidopropyl Betaine, and 65% DI Water).

Example 1d: the carrier system with Esters/Waxes/ Hydrocarbons

The carrier system is able to carry different types of esters, such as capric/caprylic triglyceride, different types of waxes, such as Phytowax olive 6L25 (INCI: hydrogenated hexyl olive esters), and different types of hydrocarbons, such as C11-13 Isoparaffin. When formulated using the general procedure as described above, clear, stable systems are obtained, as shown in table 1d.

Table 1d. Carrier system with different types of esters/waxes/hydrocarbons

| | A | B | C |
|---|---|---|---|
| D.I. Water | 46% | 46% | 46% |
| Lecithin | 5% | 5% | 5% |
| PPG-5 Ceteth-20 | 25% | 25% | 25% |
| Silicone Phosphate | 20% | 20% | 20% |
| Ester/wax/ hydrocarbon | 4% (Capric/caprylic triglyceride ) | 4% (Phytowax olive 6L25) | 4% (C11-13 Isoparaffin) |

Furthermore, all of the above formulas are dilutable in a shampoo base (25% TEA Lauryl sulfate, 10% Cocamidopropyl Betaine, and 65% DI Water). In addition, the above formulas A and B are dilutable in water.

Example 1e: the carrier system with other phospholipid

Carrier system is also clear and stable when Lecithin is replaced with another biomemetic phospholipid, such as Phospholipid EFA (INCI: Linoleamidopropyl PG-dimonium chloride phosphate) (30% active) when formulated using C11-13 Isoparaffin. See table 1e.

Table 1e. Carrier system with Phospholipid EFA carrying C11-13 Isoparaffin

| | |
|---|---|
| D.I. Water | 46% |
| Phospholipid EFA | 5% |
| PPG-5 Ceteth-20 | 25% |
| Silicone Phosphate | 20% |
| C11-13 Isoparaffin | 4% |

Furthermore, the above formula is dilutable in a shampoo base.

Example 1f: measure of conditioning effect using the carrier system

The conditioning effect of an aqueous carrier system containing the carrier composition of the present invention, as a carrier of Mineral Oil, was assessed by the wet combability method using the Instron 4444 Tensile Tester. Evaluation was carried out on bleached hair, cleansed with 15% SLES, and treated a single time with the aqueous delivery system. Specifically, swatches were treated once with 0.3g product per gram of hair. The hair was treated with the product for 1 minute and rinsed for 10 seconds. The total combing energy required to comb the hair was measured after cleansing with SLES ($W_i$), as well as after treatment ($W_f$). The percent change in combing energy was calculated using the following formula:

$$\%\ \Delta\ \text{Combing Energy} = (W_f - W_i) / (W_i) \times 100\%$$

where $W_i$ = combing energy required prior to treatment
and $W_f$ = combing energy required after treatment.
Treatments, which improve wet combability, will result in negative percent change values.

A clear, stable aqueous delivery system composed of the following formula was used to treat the hair:

| Classification | Trade Name | %wt/wt |
|---|---|---|
| Water | Water | 46 |
| Silicone Phosphate | Ultrasil PE-100 | 20 |
| Lecithin | Emulmetik 100 | 5 |
| Nonionic Surfactant | Procetyl AWS | 25 |
| Mineral Oil | Carnation White Mineral Oil | 4 |
| | | 100 |

Following a single application of the aqueous delivery system, the percent change in combing energy was -22.24%, indicating that the treated hair is significantly more conditioned. Thus, treatment with the aqueous delivery system made the hair more manageable and easier to comb by 22.24%.

Example 2: amphiphilic lipid (a) chosen from fatty monoamine compounds

General procedure: The general procedure for formulating the carrier system is as follows: First, add deionized water to the beaker and begin mixing at a moderate speed. Heat the batch to 80°C, and at 80°C, disperse Lexamine S-13 (INCI: Stearamidopropyl dimethylamine). Mix well until it is completely dissolved. Next, add Procetyl AWS (INCI: PPG-5 Ceteth-20) and Ultrasil PE-100 (silicone phosphate). Mix well. Then add the active ingredient. Mix well until formula is clear, maintaining at 80°C. Cool to room temperature. If necessary, pour at 75°C since formula may gel up around 70°C.

Example 2a: the carrier system:

Following the general procedure, a series of solutions were made as depicted in Table 2a. The results show that a complete carrier system is needed to form a clear, stable solution that contains a water insoluble silicone.

Table 2a. Experiments showing a clear, stable carrier system

| Combinations of ingredients | Appearance |
|---|---|
| Water 43g + Lexamine S-13 2g + DC 200 (300,000cst) 10g | Hazy |
| Water 43g + Procetyl AWS 30g + DC 200 (300,000cst) 10g | Hazy |
| Water 43g + Silicone Phosphate 15g + DC 200 (300,000cst) 10g | Hazy |
| Water 43g + Lexamine S-13 2g + Procetyl AWS 30g + DC 200 (300,000cst) 10g | Hazy |
| Water 43g + Lexamine S-13 2g + Silicone Phosphate 15g + DC 200 (300,000cst) 10g | Hazy |
| Water 43g + Procetyl AWS 30g + Silicone Phosphate 15g + DC 200 (300,000cst) 10g | Hazy |

(continued)

| Combinations of ingredients | Appearance |
|---|---|
| Water 43g + Lexamine S-13 2g + Procetyl AWS 30g + Silicone Phosphate 15g + DC 200 (300,000cst) 10g | Clear |

Example 2b: the carrier system with different silicones

The carrier system is able to carry different types of silicones, such as DC 200 (INCI: dimethicone), and DC 555 and DC 556 (INCI: phenyltrimethicone). When formulated using the general procedure as described above, clear, stable systems are obtained, as shown in table 2b.

Table 2b. Carrier system with different types of silicones

| | A | B | C | D | E |
|---|---|---|---|---|---|
| D.I. Water | 43% | 43% | 43% | 43% | 49% |
| Stearamidopropyl dimethylamine | 2% | 2% | 2% | 2% | 2% |
| PPG-5 Ceteth-20 | 30% | 30% | 30% | 30% | 30% |
| Silicone Phosphate | 15% | 15% | 15% | 15% | 15% |
| Silicones | 10% (DC 200 (1000cst )) | 10% (DC 200 (60,000cst)) | 10% (DC 200 (300,000cst)) | 10% (DC 556) | 4% (DC 555) |

Furthermore, the above formulas D and E are dilutable in a shampoo base (25% TEA Lauryl sulfate, 10% Cocamidopropyl Betaine, and 65% DI Water). In addition, D is dilutable in water.

Example 2c: the carrier system with different oils

The carrier system is able to carry different types of oils, such as olive oil, avocado oil, macadamia nut oil, jojoba oil, apricot kernel oil, rice bran oil, mineral oil and tea tree oil. When formulated using the general procedure as described above, clear, stable systems are obtained, as shown in table 2c.

Table 2c. Carrier system with different types of oils

| | **A** | **B** | **C** | **D** | **E** | **F** | **G** | **H** |
|---|---|---|---|---|---|---|---|---|
| D.I. Water | 21% | 21% | 21% | 21% | 21% | 21% | 43% | 43% |
| Laureth-4 | 27% | 27% | 27% | 27% | 27% | 27% | - | - |
| PPG-5 Ceteth-20 | 22% | 22% | 22% | 22% | 22% | 22% | 30% | 30% |
| Silicone Phosphate | 23% | 23% | 23% | 23% | 23% | 23% | 15% | 15% |
| Stearamidopropyl dimethylamine | 2% | 2% | 2% | 2% | 2% | 2% | 2% | 2% |
| Oils | 5% (Olive oil) | 5% (Avoca do oil) | 5% (Macadamia nut oil) | 5% (Jojoba oil) | 5% (Apricot kernel oil) | 5% (Rice bran oil) | 10% (Mineral oil) | 10% (Teatree oil) |

Furthermore, the above formulas G and H are dilutable in water and in a shampoo base (25% TEA Lauryl sulfate, 10% Cocamidopropyl Betaine, and 65% DI Water).

Example 2d: the carrier system with Esters/Waxes/Hydrocarbons

The carrier system is able to carry different types of esters, such as capric/caprylic triglyceride. When formulated using the general procedure as described above, clear, stable systems are obtained, as shown in table 2d.

Table 2d. The following chart shows that the carrier system is compatible with capric/caprylic triglyceride.

| | |
|---|---|
| D.I. Water | 20% |
| Stearamidopropyl dimethylamine | 2% |
| Laureth-4 | 26% |
| PPG-5 Ceteth-20 | 22% |
| Silicone Phosphate | 26% |
| Capric/caprylic triglyceride | 4% |

Furthermore, the above formula is dilutable in water and in a shampoo base (25% TEA Lauryl sulfate, 10% Cocamidopropyl Betaine, and 65% DI Water).

Example 2e: the carrier system with other fatty monoamines

Carrier system is also clear and stable when Lexamine S-13 is replaced with another fatty monoamine, such as PEG-15 Cocamine, PEG-2 Oleamine, Oleamidopropyl dimethylamine, and Behenamidopropyl dimethylamine with water insoluble ingredients such as mineral oil and capric/caprylic triglyceride. See table 2e.

Table 2e. Carrier system with different fatty monoamines carrying mineral oil and capric/caprylic triglyceride

| | A | B | C | D | E | F | G | H |
|---|---|---|---|---|---|---|---|---|
| D.I. Water | 20% | 20% | 20% | 20% | 20% | 20% | 20% | 20% |
| Laureth-4 | 26% | 26% | 26% | 26% | 26% | 26% | 26% | 26% |
| PPG-5 Ceteth-20 | 22% | 22% | 22% | 22% | 22% | 22% | 22% | 22% |
| Silicone Phosphate | 26% | 26% | 26% | 26% | 26% | 26% | 26% | 26% |
| Fatty monoamine | 2% (PEG-15 Cocamine) | 2% (PEG-2 Oleamine) | 2% (Oleamido-propyl dimethyl amine) | 2% (Behen-amido-propyl dimethylamine) | 2% (PEG-15 5 Cocamine) | 2% (PEG-2 Ole-amine) | 2% (Oleamido-propyl dimethylamine) | 2% (Behen-amido-propyl dimethylamine) |
| Mineral oil | 4% | 4% | 4% | 4% | - | - | - | - |
| Capric/caprylic triglyceride | - | - | - | - | 4% | 4% | 4% | 4% |

Furthermore, all of the above formulas are dilutable in a shampoo base (25% TEA Lauryl sulfate, 10% Cocamidopropyl Betaine, and 65% DI Water). In addition, formulas A, B, C, D, E, and F are dilutable in water.

Example 2f: conditioning effects of the carrier system

The conditioning effect of the carrier system, as a carrier of Dimethicone, was assessed by the wet combability method using the Instron 4444 Tensile Tester. Evaluation was carried out on bleached hair, cleansed with 15% SLES, and shampooed seven times with the carrier system formulation. Specifically, swatches were shampooed with 0.4g shampoo per gram of hair for 15 seconds and rinsed with warm water for 10 seconds. The total combing energy required to comb the hair was measured after cleansing with SLES ($W_i$), as well as after seven shampoos ($W_f$). The percent change in combing energy was calculated using the following formula:

$$\%\ \Delta\ Combing\ Energy = (W_f - W_i) / (W_i) \times 100\%$$

*where $W_i$ = combing energy required prior to 7 shampoos*
*and $W_f$ = combing energy required after 7 shampoos.*

Treatments, which improve wet combability, will result in negative percent change values.

A clear, stable shampoo composed of the following formula was used to treat the hair:

| Classification | Trade Name | %wt/wt |
|---|---|---|
| Water | Water | 54.630 |
| Silicone Phosphate | Ultrasil PE-100 | 0.500 |
| Fatty Amine | Lexamine S 13 | 0.500 |
| Anionic Surfactant | Standapol T | 35.000 |
| Dimethicone | Dow Corning 200 Fluid (60,000 cst) | 1.000 |
| Amphoteric Surfactant | Mackam 2CSF 40 CG | 5.000 |
| Nonionic Surfactant | Brij 93 VEG | 0.500 |
| Nonionic Surfactant | Arlasolve 200 | 0.500 |
| Thickener | Carbopol Ultrez 10 (2% solution) | 1.200 |
| Preservative | Methylparaben | 0.300 |
| Preservative | Glydant LTD | 0.220 |
| pH Adjuster | Triethanolamine Care | 0.550 |
| Preservative | Versene NA2 | 0.100 |

Following seven shampoos with the carrier system, the percent change in combing energy was -19.03%, indicating that the treated hair is significantly more conditioned. Thus, treatment with the carrier system made the hair more manageable and easier to comb by 19.03%.

Example 3: amphiphilic lipid (a) chosen from fatty quaternary amine compounds

General procedure: The general procedure for formulating carrier system is as follows: First, add deionized water to the beaker and begin mixing at moderate speed. Disperse in Behentrimonium chloride. Then heat the batch to 80°C, and at 80°C, add Procetyl AWS (INCI: PPG-5 Ceteth-20) and Ultrasil PE-100 (silicone phosphate). Mix well. Then add the active ingredient. Mix well until formula is clear, maintaining at 80°C. Cool to room temperature. If necessary, pour at 75°C since formula may gel up around 70°C.

Example 3a: the carrier system

Following the general procedure, a series of solutions were made as depicted in Table 3a. The results show

that a complete carrier system is needed to form a clear, stable solution that contains a water insoluble silicone.

Table 3a. Experiments showing a clear, stable carrier system

| **Combinations of ingredients** | ***Appearance*** |
|---|---|
| Water 43g + Procetyl AWS 30g + DC 200 (300,000cst) 10g | Hazy |
| Water 43g + Silicone Phosphate 15g + DC 200 (300,000cst) 10g | Hazy |
| Water 43g + Behentrimonium Chloride 2g + Procetyl AWS 30g + DC 200 (300,000cst) 10g | Hazy |
| Water 43g + Behentrimonium Chloride 2g + Silicone Phosphate 15g + DC 200 (300,000cst) 10g | Hazy |
| Water 43g + Procetyl AWS 30g + Silicone Phosphate 15g + DC 200 (300,000cst) 10g | Hazy |
| Water 43g + Behentrimonium Chloride 2g + Procetyl AWS 30g + Silicone Phosphate 15g + DC 200 (300,000cst) 10g | Clear |

Example 3b: the carrier system with different silicones

The carrier system is able to carry different types of silicones, such as DC 200 (INCI: dimethicone), and DC 555 and DC 556 (INCI: phenyltrimethicone). When formulated using the general procedure as described above, clear, stable systems are obtained, as shown in table 3b.

Table 3b. Carrier system with different types of silicones

| | A | B | C | D | E |
|---|---|---|---|---|---|
| D.I. Water | 43% | 43% | 43% | 43% | 49% |
| Behentrimonium Chloride | 2% | 2% | 2% | 2% | 2% |
| PPG-5 Ceteth-20 | 30% | 30% | 30% | 30% | 30% |
| Silicone Phosphate | 15% | 15% | 15% | 15% | 15% |
| Silicones | 10% (DC 200 (1000cst)) | 10% (DC 200 (60,000cst)) | 10% (DC 200 (300,000cst)) | 10% (DC 556) | 4% (DC 555) |

Furthermore, the above formulas D and E are dilutable in a shampoo base (25% TEA Lauryl sulfate, 10% Cocamidopropyl Betaine, and 65% DI Water). In addition, the above formula D is dilutable in water.

Example 3c: the carrier system with different oils

The carrier system is able to carry different types of oils, such as olive oil, avocado oil, macadamia nut oil, jojoba oil, apricot kernel oil, rice bran oil, mineral oil and tea tree oil. When formulated using the general procedure as described above, clear, stable systems are obtained, as shown in table 3c.

Table 3c. Carrier system with different types of oils

| | A | B | C | D | E | F | G | H |
|---|---|---|---|---|---|---|---|---|
| D.I. Water | 21% | 21% | 21% | 21% | 21% | 21% | 43% | 43% |
| Laureth-4 | 27% | 27% | 27% | 27% | 27% | 27% | - | - |
| PPG-5 Ceteth-20 | 22% | 22% | 22% | 22% | 22% | 22% | 30% | 30% |
| Silicone Phosphate | 23% | 23% | 23% | 23% | 23% | 23% | 15% | 15% |

(continued)

| | A | B | C | D | E | F | G | H |
|---|---|---|---|---|---|---|---|---|
| Behentri-monium Chloride | 2% | 2% | 2% | 2% | 2% | 2% | 2% | 2% |
| Oils | 5% (Olive oil) | 5% (Avocado oil) | 5% (Macadamia nut oil) | 5% (Jojoba oil) | 5% (Apricot kernel oil) | 5% (Rice bran oil) | 10% (Mineral oil) | 10% (Tea tree oil) |

Furthermore, the above formulas G and H are dilutable in water and in a shampoo base (25% TEA Lauryl sulfate, 10% Cocamidopropyl Betaine, and 65% DI Water).

Example 3d: the carrier system with Esters/Waxes/Hydrocarbons

The carrier system is able to carry different types of esters, such as capric/caprylic triglyceride. When formulated using the general procedure as described above, clear, stable systems are obtained, as shown in table 3d.

Table 3d. Carrier system with capric/caprylic triglyceride

| | |
|---|---|
| D.I. Water | 20% |
| Laureth-4 | 26% |
| PPG-5 Ceteth-20 | 22% |
| Silicone Phosphate | 26% |
| Behentrimonium chloride | 2% |
| Capric/caprylic triglyceride | 4% |

Furthermore, the above formula is dilutable in water and in a shampoo base (25% TEA Lauryl sulfate, 10% Cocamidopropyl Betaine, and 65% DI Water).

Example 3e: the carrier system with other fatty quats

The carrier system is also clear and stable when Behentrimonium chloride is replaced with another fatty quat, such as Incroquat Behenyl HE, Quat-22, Quat-80, and Quat-87 with water insoluble ingredients such as mineral oil and capric/caprylic triglyceride. See table 3e.

Table 3e. Carrier system with different fatty quats carrying mineral oil and capric/caprylic triglyceride

| | A | B | C | D | E | F | G | H |
|---|---|---|---|---|---|---|---|---|
| D.I. Water | 20% | 20% | 20% | 20% | 20% | 20% | 20% | 20% |
| Laureth-4 | 26% | 26% | 26% | 26% | 26% | 26% | 26% | 26% |
| PPG-5 Ceteth-20 | 22% | 22% | 22% | 22% | 22% | 22% | 22% | 22% |
| Silicone Phosphate | 26% | 26% | 26% | 26% | 26% | 26% | 26% | 26% |
| Fatty quats | 2% (Incroquat Behenyl HE) | 2% (Ceraphyl 60) | 2% (Quat-80) | 2% (Varisoft W575PG) | 2% (Incroquat Behenyl HE) | 2% (Ceraphyl 60) | 2% (Quat-80) | 2% (Varisoft W575PG) |
| Mineral oil | 4% | 4% | 4% | 4% | - | - | - | - |

(continued)

| | A | B | C | D | E | F | G | H |
|---|---|---|---|---|---|---|---|---|
| Capric/ caprylic triglyceride | - | - | - | - | 4% | 4% | 4% | 4% |
| Note:<br>Incroquat Behenyl HE = Hydroxyethyl Behenamidopropyl Dimonium Chloride<br>Ceraphyl 60 = Quat-22 60% and water 40%.<br>Quat-80 contains 5% PG.<br>Varisoft W575PG = Quat-87 75% and PG 25% | | | | | | | | |

Furthermore, all of the above formulas are dilutable in a shampoo base (25% TEA Lauryl sulfate, 10% Cocamidopropyl Betaine, and 65% DI Water). In addition, formulas A and C are dilutable in water.

Example 3f: conditioning effects of the carrier system

The conditioning effect of the carrier system, as a carrier of Avocado Oil, was assessed by the wet combability method using the Instron 4444 Tensile Tester. Evaluation was carried out on bleached hair, cleansed with 15% SLES, and treated a single time with the carrier system formulation. Specifically, swatches were treated once with 0.3g product per gram of hair. The hair was treated with the product for 1 minute and rinsed for 10 seconds. The total combing energy required to comb the hair was measured after cleansing with SLES ($W_i$), as well as after treatment ($W_f$). The percent change in combing energy was calculated using the following formula:

$$\% \Delta \text{ Combing Energy} = (W_f - W_i) / (W_i) \times 100\%$$

*where $W_i$ = combing energy required prior to treatment*
*and $W_f$ = combing energy required after treatment.*

Treatments, which improve wet combability, will result in negative percent change values.

A clear, stable carrier solution composed of the following formula was used to treat the hair:

| Classification | Trade Name | %wt/wt |
|---|---|---|
| Water | Water | 21 |
| Silicone Phosphate | Ultrasil PE-100 | 26 |
| Nonionic Surfactant | Brij 30 | 27 |
| Nonionic Surfactant | Procetyl AWS | 22 |
| Fatty Quat | Genamin KDMP | 2 |
| Avocado Oil | Avocado Oil | 4 |

Following a single application of the carrier system, the percent change in combing energy was -57.86%, indicating that the treated hair is significantly more conditioned. Thus, treatment with the carrier system made the hair more manageable and easier to comb by 57.86%.

## Claims

**1.** A composition comprising:

(a) at least one amphiphilic lipid chosen from phospholipids, fatty monoamine compounds, fatty quaternary amine compounds, and mixtures thereof;

(b) at least one nonionic surfactant having an HLB of at least 8, chosen from:

(i) alkoxylated derivatives of the following: fatty alcohols, alkyl phenols, fatty acids, fatty acid esters and fatty,acid amides, wherein the alkyl chain is in the C12-C50 range, and having from about 1 to about 110 alkoxy groups;

(ii) alkyl glycosides and polyglycosides, and alkoxylated derivatives thereof;

(iii) glyceryl esters and polyglyceryl esters, and alkoxylated derivatives thereof;
(iv) sorbitan esters, and alkoxylated derivatives thereof;
(v) and mixtures thereof;

(c) at least one anionic silicone including a silicone phosphate and/or a silicone carboxylate; and
(d) at least one water-insoluble material present in an amount of up to 10% by weight, based on the weight of the composition.

**2.** The composition of claim 1, wherein the amphiphilic lipid (a) is chosen from lecithins.

**3.** The composition of claim 1, wherein the amphiphilic lipid (a) is chosen from primary, secondary, and tertiary fatty monoamines having at least one hydrocarbon group with from 6 to 22 carbon atoms.

**4.** The composition of claim 3, wherein the amphiphilic lipid (a) is chosen from tertiary amido amines having an alkyl group with from about 12 to about 22 carbon atoms.

**5.** The composition of claim 1, wherein the amphiphilic lipid (a) is chosen from fatty quaternary amine compounds containing at least one fatty chain having from about 6 to about 22 carbon atoms.

**6.** The composition of claim 5, wherein the amphiphilic lipid (a) is behentrimonium chloride.

**7.** The composition of any preceding claim, wherein the amphiphilic lipid (a) is present in an amount of from about greater than 0 to about 30% by weight, based on the weight of the composition.

**8.** The composition of claim 7, wherein (a) is present in an amount of from about greater than 0 to about 5% by weight, based on the weight of the composition.

**9.** The composition of any preceding claim, wherein the at least one nonionic surfactant (b) is present in an amount of from about greater than 0 to about 70% by weight, based on the weight of the composition.

**10.** The composition of claim 9, wherein (b) is present in an amount of from about greater than 0 to about 20% by weight, based on the weight of the composition.

**11.** The composition of any preceding claim, wherein the at least one anionic silicone (c) is present in an amount of from about greater than 0 to about 50% by weight, based on the weight of the composition.

**12.** The composition of claim 11, wherein (c) is present in an amount of from about greater than 0 to about 15% by weight, based on the weight of the composition.

**13.** The composition of any preceding claim, wherein the at least one water-insoluble material is chosen from:

(1) silicones, oil-soluble vitamins, sunscreens, ceramides and natural oils;
(2) water-insoluble polymers, resins, and latexes, wherein the polymers and resins include those containing carboxyl moieties.

**14.** An aqueous delivery system comprising a carrier composition according to any claim 1 to 13 and an aqueous phase.

**15.** A process for making an aqueous delivery system which is both stable, and clear to slightly limpid in appearance, involving the steps of:

(a) providing a carrier composition containing: (i) at least one amphiphilic lipid chosen from phospholipids, fatty monoamine compounds, fatty quaternary amine compounds, and mixtures thereof, (ii) at least one nonionic surfactant having an HLB of at least 8, chosen from:

(i) alkoxylated derivatives of the following: fatty alcohols, alkyl phenols, fatty acids, fatty acid esters and fatty acid amides, wherein the alkyl chain is in the C12-C50 range, and having from about 1 to about 110 alkoxy groups;
(ii) alkyl glycosides and polyglycosides, and alkoxylated derivatives thereof;

(iii) glyceryl esters and polyglyceryl esters, and alkoxylated derivatives thereof;
(iv) sorbitan esters, and alkoxylated derivatives thereof;
(v) and mixtures thereof;

and, (iii) at least one anionic silicone including a silicone phosphate and/or a silicone carboxylate;
(b) providing at least one water-insoluble ingredient in an amount of up to 10% by weight, based on the weight of the composition;
(c) optionally, heating the composition of step (a) to form a heated mixture;
(d) adding (b) to either step (a), step (c) or both step (a) and (c);
(e) adding an aqueous solution to either (c) or (d) to form a diluted mixture; and
(f) cooling the diluted mixture to form the aqueous delivery system.

**16.** A process for treating a keratinous substrate comprising contacting the keratinous substrate with a composition according to any claim 1 to 14.

**17.** The process of claim 16 wherein the keratinous substrate is hair.

**18.** A personal care composition comprising the composition of any claim 1 to 14.

**19.** The composition of claim 18 wherein the composition is chosen from a shampoo, a conditioner, a deep treatment, or a combination shampoo and conditioner.

## Patentansprüche

**1.** Zusammensetzung, die enthält:

(a) mindestens ein amphiphiles Lipid, das unter den Phospholipiden, Fettmonoaminen, quartären Fettaminen und deren Gemischen ausgewählt ist;
(b) mindestens einen nichtionischen grenzflächenaktiven Stoff, der einen HLB-Wert von mindestens 8 aufweist und der ausgewählt ist unter:

(i) alkoxylierten Derivaten der folgenden Verbindungen: Fettalkoholen, Alkylphenolen, Fettsäuren, Fettsäureestern und Fettsäureamiden, wobei die Alkylkette 12 bis 50 Kohlenstoffatome aufweist und etwa 1 bis etwa 110 Alkoxygruppen besitzt;
(ii) Alkylglycosiden und Alkylpolyglycosiden, und deren alkoxylierten Derivaten;
(iii) Glycerylestern und Polyglycerylestern, und deren alkoxylierten Derivaten;
(iv) Sorbitanestern und deren alkoxylierten Derivaten; und
(v) deren Gemischen;

(c) mindestens ein anionisches Silicon, der ein Siliconphosphat und/oder ein Siliconcarboxylat umfasst; und
(d) mindestens ein wasserunlösliches Material, das in einer Menge von bis zu 10 Gew.-%, bezogen auf das Gesamtgewicht der Zusammensetzung, enthalten ist.

**2.** Zusammensetzung nach Anspruch 1, wobei das amphiphile Lipid (a) unter den Lecthinen ausgewählt ist.

**3.** Zusammensetzung nach Anspruch 1, wobei das amphiphile Lipid (a) unter den primären, sekundären und tertiären Fettmonoaminen mit mindestens einer Kohlenwasserstoffgruppe mit 6 bis 22 Kohlenstoffatomen ausgewählt ist.

**4.** Zusammensetzung nach Anspruch 3, wobei das amphiphile Lipid (a) unter den tertiären Amidoaminen ausgewählt ist, die eine Alkylgruppe mit etwa 12 bis 22 Kohlenstoffatomen aufweist.

**5.** Zusammensetzung nach Anspruch 1, wobei das amphiphile Lipid (a) unter den quartären Fettaminen ausgewählt ist, die mindestens eine Fettkette mit etwa 6 bis etwa 22 Kohlenstoffatomen aufweisen.

**6.** Zusammensetzung nach Anspruch 5, wobei das amphiphile Lipid (a) das Behentrimonium Chloride ist.

**7.** Zusammensetzung nach einem der vorhergehenden Ansprüche, wobei das amphiphile Lipid (a) in einer Menge

von ungefähr größer als 0 bis etwa 30 Gew.-%, bezogen auf das Gesamtgewicht der Zusammensetzung, vorliegt.

**8.** Zusammensetzung nach Anspruch 7, wobei (a) in einer Menge von ungefähr größer als 0 bis etwa 5 Gew.-%, bezogen auf das Gesamtgewicht der Zusammensetzung, vorliegt.

**9.** Zusammensetzung nach einem der vorhergehenden Ansprüche, wobei der zumindest eine nichtionische grenzflächenaktive Stoff (b) in einer Menge von ungefähr größer als 0 bis etwa 70 Gew.-%, bezogen auf das Gesamtgewicht der Zusammensetzung, enthalten ist.

**10.** Zusammensetzung nach Anspruch 9, wobei (b) in einer Menge von ungefähr größer als 0 bis etwa 20 Gew.-%, bezogen auf das Gesamtgewicht der Zusammensetzung, enthalten ist.

**11.** Zusammensetzung nach einem der vorhergehenden Ansprüche, wobei das zumindest eine anionische Silicon (c) ein einer Menge von ungefähr größerals 0 bis etwa 50 Gew.-%, bezogen auf das Gesamtgewicht der Zusammensetzung, enthalten ist.

**12.** Zusammensetzung nach Anspruch 11, wobei (c) in einer Menge von ungefähr größer als 0 bis etwa 15 Gew.-%, bezogen auf das Gesamtgewicht der Zusammensetzung, enthalten ist.

**13.** Zusammensetzung nach einem der vorhergehenden Ansprüche, wobei das zumindest eine in Wasser unlösliche Material ausgewählt ist unter:

(1) Siliconen, öllöslichen Vitaminen, Sonnenschutzfiltern, Ceramiden und natürlichen Ölen;
(2) in Wasser unlöslichen Polymeren, Harzen und Latices, wobei die Polymere und Harze solche Verbindungen mit Carboxyeinheiten umfassen.

**14.** Wässriges Abgabesystem, das eine Trägerzusammensetzung nach einem der Ansprüche 1 bis 13 und eine wässrige Phase enthält.

**15.** Verfahren zur Herstellung eines wässrigen Abgabesystems, das stabil ist und gleichzeitig klar bis leicht trübe aussieht, mit den folgenden Schritten:

(a) Bereitstellen einer Trägerzusammensetzung, die enthält:

(i) mindestens ein amphiphiles Lipid, das ausgewählt ist unter den Phospholipiden, Fettmonoaminen, quartären Fettaminen und deren Gemischen,
(ii) mindestens einen nichtionischen grenzflächenaktiven Stoff, der einen HLB-Wert von mindestens 8 aufweist und der ausgewählt ist unter:

- alkoxylierten Derivaten der folgenden Verbindungen: Fettalkoholen, Alkylphenolen, Fettsäuren, Fettsäureestern und Fettsäureamiden, wobei die Alkylkette 12 bis 50 Kohlenstoffatome aufweist und etwa 1 bis etwa 110 Alkoxygruppen besitzt;
- Alkylglycosiden und Alkylpolyglycosiden und deren alkoxylierten Derivaten;
- Glycerylestern und Polyglycerylestern und deren alkoxylierten Derivaten;
- Sorbitanestern und deren alkoxylierten Derivaten; und
- deren Gemischen;

und (iii) mindestens ein anionisches Silicon, der ein Siliconphosphat und/oder ein Siliconcarboxylat umfasst;

(b) Bereitstellen mindestens eines in Wasser unlöslichen Bestandteils, in einer Menge von bis zu 10 Gew.-%, bezogen auf das Gesamtgewicht der Zusammensetzung;
(c) optional Erwärmen der Zusammensetzung aus Schritt (a), um ein erwärmtes Gemisch zu bilden;
(d) Zugabe von (b) entweder zu Schritt (a), Schritt (c) oder sowohl Schritt (a) als auch Schritt (c);
(e) Zugabe einer wässrigen Lösung entweder zu (c) oder (d), um ein verdünntes Gemisch zu bilden; und
(f) Abkühlen des verdünnten Gemisches, um ein wässriges Abgabesystem zu bilden.

**16.** Verfahren zur Behandlung einer Keratinsubstanz, das umfasst, die Keratinsubstanz mit einer Zusammensetzung nach einem der Ansprüche 1 bis 14 in Kontakt zu bringen.

**17.** Verfahren nach Anspruch 16, wobei es sich bei der Keratinsubstanz um Haare handelt.

**18.** Körperpflegezusammensetzung, die eine Zusammensetzung nach einem der Ansprüche 1 bis 14 enthält.

**19.** Zusammensetzung nach Anspruch 18, wobei die Zusammensetzung ausgewählt ist unter Haarwaschmitteln, Konditionern, tief Behandlungsmitteln oder Kombinationen von Haarwaschmitteln und Konditionern.

**Revendications**

**1.** Composition comprenant :

a) au moins un lipide amphiphile, choisi parmi les phopholipides, les monoamines grasses, les dérivés quaternaires d'amines grasses, ainsi que les mélanges de tels composés ;
b) au moins un tensioactif non-ionique présentant un rapport hydrolipophile d'au moins 8, choisi parmi les suivants :

i) les dérivés alcoxylés des composés suivants : alcools gras, alkyl-phénols, acides gras, esters d'acide gras et amides d'acide gras, dans lesquels la chaîne alkyle comporte de 12 à 50 atomes de carbone et qui comportent d'environ 1 à environ 110 groupes alcoxy,
ii) les alkyl-glycosides et alkyl-polyglycosides, ainsi que leurs dérivés alcoxylés,
iii) les esters de glycéryle et les esters de polyglycéryle, ainsi que leurs dérivés alcoxylés,
iv) les esters de sorbitane, ainsi que leurs dérivés alcoxylés,
v) et les mélanges de tels composés ;

c) au moins une silicone anionique incluant une silicone phosphatée et/ou une silicone carbonylée ; et
d) au moins une substance insoluble dans l'eau, présente en une quantité pouvant aller jusqu'à 10 % en poids, par rapport au poids de la composition.

**2.** Composition conforme à la revendication 1, dans laquelle le lipide amphiphile (a) est choisi parmi les lécithines.

**3.** Composition conforme à la revendication 1, dans laquelle le lipide amphiphile (a) est choisi parmi les monoamines grasses, primaires, secondaires ou tertiaires, qui comportent au moins un groupe hydrocarboné ayant d'environ 6 à environ 22 atomes de carbone.

**4.** Composition conforme à la revendication 3, dans laquelle le lipide amphiphile (a) est choisi parmi les amido-amines tertiaires qui comportent un groupe allyle ayant d'environ 12 à environ 22 atomes de carbone.

**5.** Composition conforme à la revendication 1, dans laquelle le lipide amphiphile (a) est choisi parmi les dérivés quaternaires d'amines grasses qui comportent au moins une chaîne grasse ayant d'environ 6 à environ 22 atomes de carbone.

**6.** Composition conforme à la revendication 5, dans laquelle le lipide amphiphile (a) est du chlorure de béhentrimonium.

**7.** Composition conforme à l'une des revendications précédentes, dans laquelle le lipide amphiphile (a) se trouve en une quantité qui représente d'à peu près plus de 0 % à environ 30 % en poids, par rapport au poids de la composition.

**8.** Composition conforme à la revendication 7, dans laquelle le composant (a) se trouve en une quantité qui représente d'à peu près plus de 0% à environ 5 % en poids, par rapport au poids de la composition.

**9.** Composition conforme à l'une des revendications précédentes, dans laquelle le tensioactif non-ionique (b) se trouve en une quantité qui représente d'à peu près plus de 0 % à environ 70 % en poids, par rapport au poids de la composition.

**10.** Composition conforme à la revendication 9, dans laquelle le composant (b) se trouve en une quantité qui représente d'à peu près plus de 0 % à environ 20 % en poids, par rapport au poids de la composition.

**11.** Composition conforme à l'une des revendications précédentes, dans laquelle la silicone anionique (c) se trouve en

une quantité qui représente d'à peu près plus de 0 % à environ 50 % en poids, par rapport au poids de la composition.

**12.** Composition conforme à la revendication 11, dans laquelle le composant (c) se trouve en une quantité qui représente d'à peu près plus de 0 % à environ 15 % en poids, par rapport au poids de la composition.

**13.** Composition conforme à l'une des revendications précédentes, pour laquelle la substance insoluble dans l'eau est choisie parmi :

1) les silicones, vitamines oléosolubles, écrans solaires, céramides et huiles naturelles ;
2) les polymères, résines et latex insolubles dans l'eau, y compris, parmi les polymères et résines, ceux et celles qui portent des groupes carboxyle.

**14.** Système aqueux de distribution, comprenant une composition véhicule, conforme à l'une des revendications 1 à 13, et une phase aqueuse.

**15.** Procédé de préparation d'un système de distribution aqueux qui soit à la fois stable et d'aspect transparent à légèrement limpide, lequel procédé comprend les étapes suivantes :

a) prendre une composition véhicule contenant :

i) au moins un lipide amphiphile, choisi parmi les phopholipides, les monoamines grasses, les dérivés quaternaires d'amines grasses, ainsi que les mélanges de tels composés,
ii) au moins un tensioactif non-ionique présentant un rapport hydrolipophile d'au moins 8, choisi parmi les suivants :

- les dérivés alcoxylés des composés suivants : alcools gras, alkyl-phénols, acides gras, esters d'acide gras et amides d'acide gras, dans lesquels la chaîne alkyle comporte de 12 à 50 atomes de carbone et qui comportent d'environ 1 à environ 110 groupes alcoxy,
- les alkyl-glycosides et alkyl-polyglycosides, ainsi que leurs dérivés alcoxylés,
- les esters de glycéryle et les esters de polyglycéryle, ainsi que leurs dérivés alcoxylés,
- les esters de sorbitane, ainsi que leurs dérivés alcoxylés,
- et les mélanges de tels composés,

iii) et au moins une silicone anionique incluant une silicone phosphatée et/ou une silicone carboxylée ;

b) prendre au moins un ingrédient insoluble dans l'eau en une quantité pouvant aller jusqu'à 10 % en poids, par rapport au poids de la composition ;
c) en option, chauffer la composition résultant de l'étape (a) pour en faire un mélange chauffé ;
d) ajouter le produit de l'étape (b) à celui de l'étape (a), de l'étape (c) ou des deux étapes (a) et (c);
e) ajouter une solution aqueuse au résultat de l'étape (c) ou (d), pour en faire un mélange dilué ;
f) et faire refroidir ce mélange dilué, pour en faire un système de distribution aqueux.

**16.** Procédé de traitement d'un substrat kératinique, qui comporte le fait de mettre le substrat kératinique en contact avec une composition conforme à l'une des revendications 1 à 14.

**17.** Procédé conforme à la revendication 16, dans lequel le substrat kératinique est une chevelure.

**18.** Composition de soin personnel, comprenant une composition conforme à l'une des revendications 1 à 14.

**19.** Composition conforme à la revendication 18, laquelle composition est choisie parmi un shampooing, un soin de conditionnement, un traitement en pro-fondeur, ou une combinaison d'un shampooing et d'un un soin de conditionnement.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5248783 A
- US 5739371 A